(19) Europäisches Patentamt
European Patent Office
Office européen des brevets

(11) Publication number: 0 026 932
B1

(12) EUROPEAN PATENT SPECIFICATION

(45) Date of publication of patent specification: 15.01.86

(21) Application number: 80106015.3

(22) Date of filing: 03.10.80

(51) Int. Cl.[4]: C 08 F 2/26, C 08 F 246/00 // (C08F246/00, 220:06)

# (54) Aqueous emulsion comprising the reaction products of a vinyl acid monomer and a second copolymerisable monomer.

(30) Priority: 03.10.79 US 81367
03.10.79 US 81322

(43) Date of publication of application:
15.04.81 Bulletin 81/15

(45) Publication of the grant of the patent:
15.01.86 Bulletin 86/03

(84) Designated Contracting States:
BE DE FR GB IT NL

(56) References cited:
EP-A-0 005 221
BE-A- 630 043
US-A-4 148 746

(73) Proprietor: GAF CORPORATION
140 West 51st Street
New York New York 10020 (US)

(72) Inventor: Chakrabarti, Paritosh M.
3 Alicia Court
Cedar Grove New Jersey 07009 (US)
Inventor: Kirchner, Darrell G.
SR Box 18-12
Glenwood New Jersey 07418 (US)

(74) Representative: Weinhold, Peter, Dr. et al
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

EP 0 026 932 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Surfactant sold under the trademark Triton® by Rohm & Haas Co. and Alipal® SE-463 by GAF Corp. are alkyl aryl polyether sulfonate, sodium salts. These sulfonates offer many diversified surfactant properties not commonly encountered in a single compound. They out perform comparable sulfates in offering resistance to decomposition under both highly acidic and basic conditions, thus being suitable in metal cleaning applications for either acid pickling baths or alkaline cleaning formulations. Their excellent compatibility with alkaline detergent builders and their excellent emulsifying character with fats, greases, oils and gelatins afford many applications possibilities in lime soap formulation, the film coating industry, as dye leveling agents, post-latex stabilizers and in emulsion polymerization. A major application for this type of surfactant is a shampoo base for the cosmetics industry where it finds wide applicability because of its detergent, lathering and solubilizing properties.

However, it has now been found that in certain applications, as for example the production of high acid level latexes, such as, acrylic and methacrylic acid copolymer latexes containing in excess of 10% acrylic acid, coagulation occurs. The problem becomes most acute when attempting to store the latex for over a week. The low shelf life of these latexes renders them unusable in many industrial applications because of the commonly encountered need to store the latex before further processing can be commenced.

The production of high solids content latexes has long been considered to be desirable, the advantage being the maximizing use of production equipment and the minimization of the quantity of water which must be shipped in a latex. High solids content emulsions tend to coagulate immediate or become unstable after relatively short periods of storage. US—A—3,637,563 which is directed to the production of high solid aqueous polymer emulsions suggests the use of surfactants such as, alkylphenoxy poly(ethyleneoxy) ethanols, as well as the use of higher molecular weight sulfates and sulfonates. Among the materials specifically referred to in the aforenoted patent are alkylphenoxy poly(ethyleneoxy) ethanols which contain from about 30 to about 100 ethyleneoxy units, and typical anionic surfactants, e.g., an ethoxylated higher fatty acid which has also been sulfonated. In order to produce a stable emulsion employing of the disclosure of 3,637,563, it is necessary to employ one or more protective colloids. Included among such materials are ether linkage containing protective colloids, such as hydroxy methyl cellulose, hydroxy ethyl cellulose, ethyl hydroxy ethyl cellulose, carboxy methyl cellulose, ethoxylated starch derivatives, and the like. Other protective colloid forming substances, i.e., those containing no ether linkages, are also disclosed as being usable, either alone or together with the aforementioned ether linkage-containing materials.

It has now been found that high acid content latexes can be produced through the use of novel polyethyleneoxy sulfonates surfactants in accordance with the present invention. An aqueous emulsion is produced from the reaction product of a vinyl acid monomer and at least a second copolymerizable monomer, said vinyl acid monomer being present in an amount equal to 5% to 50% by weight based on total weight of said vinyl acid monomer and said at least a second copolymerizable monomer. The surfactant being present in an amount equal to 0.1 to 10 weight percent based on the total weight of said monomer is represented by the formula:

$$RO\text{-}(CH_2CH_2O)_{n-1}CH_2CH_2SO_3Me$$

wherein:

R is an alkyl having 10 to 14 carbon atoms or a phenyl group, which is substituted by an alkyl group of 6 to 18 carbon atoms or 2 alkyl groups in which the first alkyl group contains 6 to 18 carbon atoms and the second alkyl group contains 6 to 12 carbon atoms;

n is at least 3; and

Me is $NH_4$, Na, Li or K, with the proviso that Me=Na when R is an alkyl group with 10 to 14 carbon atoms.

In order that those skilled in the art can more fully understand the present invention, the following detailed description and examples are provided. These examples are intended solely for the purpose of illustrating the invention, and are not to be construed as expressing limitations unless so set forth in the appended claims. All parts and percentages are by weight, unless otherwise stated.

Useful surfactants are:

Alfol 1012+3 E.O. Sulfonate, R is a straight chain alkyl residue having an average of between 10 and 12 carbons and n is 3.

Alfol 12+4 E.O. Sulfonate, R is a straight chain alkyl residue having an average of between 10 and 12 carbon atoms and n is 4.

Alfol 12+E.O. Sulfonate, R is a straight chain alkyl residue having 12 carbons and n is 4.

Alfol 1214, R is straight chain alkyl residue having an average of 12 to 14 carbons and n is 6.

In oxotridecyl+4.2 E.O. Sulfonate, $R_2$ is an oxotridecyl group and n averages about 4.2.

Emulphogene BC-720, R is $C_{13}H_{27}$; and n is greater than about 9.

Igepal CO-630, R is nonyl phenyl and n is 9.

Igepal CO-850, R is nonyl phenyl and n is 20.

Igepal CO-890, R is nonyl phenyl and n is 40.

In the above mentioned Emulphogene and the Igepals $SO_3Me$ in the above formula has to be replaced by the OH group.

Emulphogene® and Igepal® are registered trademarks of GAF Corporation and Alfol® is a trademark of Continental Oil Company.

It is seen from the comparison in Table I that the use of various sulfonate surfactants in the emulsion polymerization methacrylic acid copolymers produces latexes having shelf-lives in excess of several months, whereas an analogous sulfate surfactant was unable to give the desired results.

Basically, the technology of producing the sulfonates involves initially transforming the terminal alcohol functionality of a nonionic surfactant to a terminal chloride using thionyl chloride, or other chlorinating agent as follows:

1) $$RO(CH_2CH_2O)_nCH_2CH_2OH \xrightarrow{SOCl_2} RO(CH_2CH_2O)_nCH_2CH_2Cl$$

The chloride terminated material is then converted to the sodium sulfonate, using sodium sulfite, as follows:

2) $$RO(CH_2CH_2O)_nCH_2CH_2Cl \xrightarrow{Na_2SO_3} RO(CH_2CH_2O)_nCH_2CH_2SO_3Na$$

Looking now in greater detail to the chemistry of the production of the surfactants used in the present invention it is noted that the synthetic approach to the production of poly ether sulfonate type surfactants involves the reaction of chlorine capped nonionics with sodium sulfite, as disclosed in US—A—2,115,192.

$$R(OC_2H_4)_nCl + Na_2SO_3 \xrightarrow[H_2O]{OH—} R(OC_2H_4)_nSO^-{}_3Na^+ + NaCl$$

The reaction requires high temperature—155—170°C being the most desirable range—and pressures of about 6 to 9 bar (70—115 psig). To attain over 90% conversion of the chlorine terminated nonionics, reaction times of about 20 hours are typical. The rate of sulfonation appears to be 100 to 500 times the rate of hydrolysis under proper reaction conditions such that good conversions can be expected with most nonionic types under the reaction conditions to be defined in this report. The competing hydrolysis reaction results in a portion of OH terminated nonionics being present in the reaction product as depicted below.

$$R(OC_2H_4)_nCl \xrightarrow[\Delta]{OH^-/H_2O} R(OC_2H_4)_nOH \text{ (Hydrolysis)}$$

The sulfonate method of preparation is well-known to those skilled in the art as represented by patents such as US—A—4,091,014, 2,209,911 and 2,148,432.

US—A—4,091,014 is noted to disclose the manufacture of ether sulfonates, by sulfonating alcohols such as:

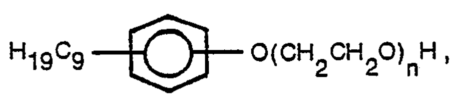

where n is an integer such as 3, 4 and 5. US—A—2,209,911 discloses the production of alkylated phenoxy ether halides, while US—A—2 148 432 discloses the method of producing alkyl phenoxy polyethylene oxide sulfonates from the corresponding alkyl phenoxy poly ethoxy halide. It should thus be evident that the sodium salts of polyether sulfonates used in the present invention can be made in accordance with the teachings of the aforenoted patents.

Although a comparison of sulfonate conversion by methylene blue activity may not be accurate through the Igepal CO series, the presence of unreacted sulfite indicates a reduced reactivity of the chlorine terminated nonionic with increasing chain length whatever the product distribution from the chloride might be. Inclusion of a more reactive halogen (NaBr, NaI) can be employed to improve the nonionic reactivity by in situ halogen exchange.

$$R(OC_2H_4)_nCl \xrightarrow[—NaCl]{NaBr} R(OC_2H_4)_nBr \xrightarrow{Na_2SO_3} R(OC_2H_4)_nSO_3Na + NaBr$$

Alternatively, the charge of $Na_2SO_3$ can be increased to increase the reactive collison frequency. In the case of Igepal CO-850 at 50% excess of sulfite would result in an increase in salt content in the product of only 2.0%, thus increasing the salt content in the final product but to a tolerable level.

The sulfonate functionality has much less tendency to hydrolize in solutions of low pH than does an analogous surfactant with a sulfate functionality.

3) $R'CH_2SO_3Na \xrightarrow{H+} $ Stable (sulfonate)

4) $R'CH_2OSO_3Na \xrightarrow{H+} RCH_2OH$ (Sulfate decomposition)

(R'=organic residue)

The ether sulfonates are noted to contain both an ethoxylated nonionic portion and a sulfonic acid anionic portion.

Preparation of surfactants:

1. Preparation of Alfol 1012+3 EO ether sulfonate

a. Chlorination

A 1 liter r.b. flask equipped with mechanical stirrer, thermometer, dropping funnel and condenser adapted to a caustic scrubber was charged with 600 g of Alfol 1012+3 E.O. (2.0 moles). To it were added 286 g of thionyl chloride (2.4 moles) maintaining the reaction mixture 55°C during addition. The mixture was then heated to 100°C for 1 hour, the condenser removed and heated to 100—110°C with an air purge for 2-1/2 hours.

The appearance of the chloride was improved by the addition of 3 g of 30% $H_2O_2$ at 80°C and heating to 100—110°C for 15 minutes (VCS 15 initial; VCS 9.5 final). Analysis: Cl 11.67/11.2 (actual/theory).

b. Sulfonation

A charge of 420 gms of the Alfol 1012+3 EO chloride (1.32 moles) was combined with 175 g of $Na_2SO_3$ (1.39 moles), 1000 ml of distilled water and 4.5 g of 50% NaOH in a 3.8 l (1 gallon) autoclave and reacted at 160—5°C for 10 hours. Stirring speed was 1750 rpm. The product was cooled to 40°C was discharged. It was a viscous, light yellow liquid (VCS 4+) having a methylene blue (M.B.) activity of 25.7% (80.8%) based on a molecular weight of 386, a chloride content of 5.27% and a pH of 5.3 (20% solution). The excess sulfite was removed by adding 16.5 g of 30% $H_2O_2$ at 30—40°C and the pH adjusted to 7.2 with 9.5 g of 50% NaOH. The final product properties include 1.83% $Na_2SO_4$, 4.65% NaCl and 36.2% total solids.

2. Preparation of Alfol 12+4 EO ether sulfonate

a. Chlorination

A 1 liter round bottom flask equipped with mechanical stirrer, thermometer, dropping funnel and condenser adapted to a caustic scrubber was charged with 500 g (1.38 moles) of Alfol 12+4 EO. To it was added 205 g (1.73 moles) of thionyl chloride at a rate which conveniently maintained the temperature below 55°C. The mixture was then heated to 100—105°C for 4 hours and the disappearance of the IR absorption at 3500 $cm^{-1}$ (OH) monitored. When the OH band had been removed, the condenser was removed and the chlorosulfite heated to 100—110°C with an air purge to remove the $SO_2$ formed. The air purging and heating were continued for 4 hours until the inorganic chloride had dropped to 0.07%. Analysis: Cl, 9.34/9.33 (actual/theory).

b. Sulfonation

A charge of 440 gms of the Alfol 12+40 EO chloride (1.16 moles) was combined with 158 g of $Na_2SO_3$ (1.25 moles), 880 g of distilled water and 8 g of 50% NaOH in a 3.8 l (1 gallon) autoclave and reacted at 160—5°C for 20 hours. Stirring speed was 1750 rpm. The product was cooled to 60°C and discharged. It was a viscous, clear liquid (VCS 2+) which solidified on standing overnight (it has a m.pt. of 28°C and if diluted to a M.B. activity of 19% remains a liquid at room temperature). Preliminary data indicated a methylene blue activity of 28.8% (72.7%) based on a molecular weight of 448, and a chloride content of 4.32% (84%). The excess sulfite was removed by adding 22 g $H_2O_2$ stirring at room temperature (a starch-iodine test confirmed the absence of sulfite) and the pH was adjusted to 7.3 with 4.5 g of 50% NaOH. Final product properties include 1.87% $Na_2SO_4$, 2.95% NaCl, 40.2% total solids, 4.76% nonionics.

3. Preparation of Alfol 1214+6 EO ether sulfonate

a. Chlorination

A 2 l. flask equipped with mechanical stirrer, thermometer, dropping funnel and condenser adapted to a caustic scrubber was charged with 752 g of Alfol 1214+6 EO (2.0 moles). To it were added 286 g of thionyl chloride (2.4 moles) maintaining the reaction temperature below 55°C. When the addition was completed, the reaction mixture was heated to 100°C for 1/2 hour, the condenser removed and heating at 100—110°C continued for three hours under an air purge (subsurface) to remove $SO_2$. The appearance of the chloride was improved by the addition of 40 g of 30% $H_2O_2$ added at 65°C followed by heating to 90°C for 15 min. (VCS 13.5 initial; VCS 7 final).

Analysis: Cl 8.61/9.00 (actual/theory).

b. Sulfonation

A charge of 711 g of the Alfol 1214+4 EO chloride (1.80 moles) was combined with 238 g of $Na_2SO_3$ (1.89 moles), 1500 ml distilled water and 12 g of 50% NaOH in a 3.8 l (1 gallon) autoclave and reacted at 160—5°C for 20 hours at 1750 rpm.

The product was cooled to 40°C and discharged. It was a layer system initially (viscous yellow liquid—top layer hazy, bottom layer clear) which solidified on standing. As such it had a M.B. activity of 24.4% (72.2%) based on a molecular weight of 462, a chloride content of 4.68% (101%) and a pH of 6.6 (20% solution). The excess sulfite was removed by the addition of 54 g of 30% $H_2O_2$ and the pH was adjusted to 7.2 (20% solution) by addition of 10 g 50% caustic. The final product properties include 2.68% $Na_2SO_4$, 4.50% NaCl and 39.5% total solids. The sample is a white solid at room temperature at this concentration. Dilution with distilled water to a M.B. activity of 13.3% affords a clear, yellow, liquid product.

4. Preparation of oxo tridecyl+4.2 EO ether sulfonate

a. Chlorination

A 2 l. flask equipped with mechanical stirrer, thermometer, dropping funnel and condenser adapted to a caustic scrubber was charged with 800 g of Emulphogene BC-420 (2.4 moles). To it was added 343 g of thionyl chloride (2.88 moles) maintaining the temperature below 55°C during addition. The reaction mixture was then heated to 100—105°C continued for 1-1/2 hours with an air purge (subsurface) to remove $SO_2$. The appearance of the chloride was improved by the addition of 30 g of 30% $H_2O_2$ at 70°C and heating to 100°C for 1/2 hour (VCS 18 initial; VCS 12 final). The final inorganic chloride was 0.1%. Analysis: Cl 9.91/10.1 (actual/theory).

b. Sulfonation

A charge of 445 g of the Emulphogene BC-420 chloride (1.27 moles) was combined with 168 g of $Na_2SO_3$ (1.33 moles), 1000 ml of distilled water and 10 g of 50% NaOH in a 3.8 l (1 gallon) autoclave and reacted at 160—5°C for 20 hours at 1750 rpm. The product was cooled to 40°C and discharged. It was a viscous gelatenous material having a M.B. activity of 22.9% (70.1%) based on a molecular weight of 418, an inorganic chloride content of 4.68% and a pH of 6.7 (20% solution). The excess sulfite was removed by the addition of 72 g of 30% $H_2O_2$ and the pH adjusted to 7.5 with 9 g of 50% NaOH. The final product properties include 2.36% $Na_2SO_4$, 4.41% NaCl and 34.6% total solids. It offers a clear yellow solution with 25% ethanol. Dilutions to 13% M.B. activity with water, also provide a clear liquid above 70°C which, however, clouds on slight cooling.

5. Preparation of nonylphenol+9 EO ether sulfonate

a. Chlorination

A 3 l. flask equipped with mechanical stirrer, thermometer, dropping funnel and condenser adapted to a caustic scrubber was charged with 2000 g of Igepal CO-630 (3.28 moles). To it were added 540 g of thionyl chloride (4.54 moles) maintaining the reaction temperature below 60°C. When the addition was completed the reaction mixture was heated to 120°C for 1/2 hour at which time an IR revealed no OH band at 3500 $cm^{-1}$. The condenser was removed and heating at 100—110°C continued with removal of $SO_2$ by a subsurface nitrogen purge. The heating was continued for a total of 7 hours whereupon conversion to the chloride was essentially complete. The appearance of the chloride was improved by bleaching with 50 g of 30% $H_2O_2$ at 70—90°C for 15 minutes (VCS 18 initial, VCS 13 final). Analysis: Cl 5.51/5.63 (actual/theory).

b. Sulfonation

A charge of 638 g of the Igepal CO-630 chloride (1.0 mole) was combined with 131 g of $Na_2SO_3$ (1.0 mole), 1200 ml of distilled water and 6.0 g of 50% NaOH in a 3.8 l (1 gallon) autoclave and reacted at 160—5°C for 20 hours at 1750 rpm. The product was cooled to 40° and discharged. It was a very viscous clear yellow liquid having a M.B. activity of 25.5% (71.4%) based on a molecular weight of 698, a chloride content of 2.98% (101%) and a VCS of 2. The excess sulfite was removed by the addition of 16 g of 30% $H_2O_2$ and the pH adjusted to 7.4 with 9 g of 50% NaOH. The final product properties include 1.48% $Na_2SO_4$, and 40.3% total solids.

6. Preparation of nonylphenol and 20 EO ether sulfonate

The procedure employed for producing the nonylphenol 9 EO Ether sulfonate can be followed using Igepal CO-850 and thionyl chloride in a 1 to 1.5 mole ratio in the chlorination procedure and a 1 to 1 Igepal CO-850 chloride to sodium sulfite mole ratio in the sulfonation procedure.

7. Preparation of nonylphenol and 40 EO ether sulfonate

The procedure for producing the nonylphenol 9 E.O. ether sulfonate can be followed, using Igepal CO-890 in approximately a 1 to 1.5 mole ratio with thionyl chloride. The sulfonation step can employ the resultant chloride of Igepal CO-890 in a 1 to 4 mole ratio with sodium sulfite.

Examples

1.) Preparation of a butyl acrylate-styrene-methacrylic acid (50-25-25) terpolymer latex

To a 2 liter resin kettle was added 9.8 g. of the desired surfactant (based on 100% methylene blue activity), 291.5 g water (including water present in the surfactant), and 0.5 g potassium persulfate. Nitrogen purge was started immediately. To the kettle was added 37.5 g butyl acrylate, 19.0 g styrene, and 19.0 g methacrylic acid. The kettle was then heated with stirring to 75—80°C. While the kettle was being heated to the final temperature, a pre-emulsion was prepared which contained 2.8 g surfactant, 139.2 g water, 0.5 g potassium persulfate, 112.5 g butyl acrylate, 56.0 g styrene, and 56.0 g methacrylic acid. The pre-emulsion was transferred to the pressure-equalizing addition funnel. The funnel was fitted with a mechanical stirrer, and was attached to the kettle. When the kettle reached 75—80°C, the stirred pre-emulsion was added dropwise over a 3 hour period. The 75—80°C range was maintained throughout. When addition was complete, heat was continued an additional 40 minutes to insure complete reaction. The kettle was then cooled to room temperature and the latex was discharged through a 0.25 mm sieve.

2.) Preparation of a butyl acrylate-styrene methacrylic acid (30-30-40) terpolymer latex

To a 2 liter resin kettle was added 9.8 g of the desired surfactant (based on 100% methylene blue activity), 291.5 g water (including water present in the surfactant), and 0.5 g potassium persulfate. Nitrogen purge was started immediately. To the kettle was added 28.3 g butyl acrylate, 28.3 g styrene, and 37.75 g methacrylic acid. The kettle was then heated with stirring to 75—80°C. While the kettle was being heated to the final temperature, a pre-emulsion was prepared which contained 2.8 g surfactant, 139.2 g water, 0.5 g potassium persulfate, 84.2 g butyl acrylate, 84.2 g styrene and 112.25 g methacrylic acid. The pre-emulsion was placed in a pressure-equalizing additional funnel. The funnel was fitted with a mechanical stirrer, and was attached to the kettle. When the kettle reached 75—80°C the stirred pre-emulsion was added dropwise over a 3 hour period. The 75—80°C range was maintained throughout. When addition was complete, heat was continued an additional 40 minutes to insure complete reaction. The kettle was cooled to room temperature and the latex was discharged through a 0.25 mm sieve.

3.) Preparation of methacrylic acid-butyl acrylate (60-40) latex

To a 2 liter resin kettle was added 260.5 g water, 40.75 g Igepal CO-630 sulfonate (24.5% methylene blue activity), and 0.5 g potassium persulfate. Nitrogen purge was started immediately. To the kettle was added 45.6 g methacrylic acid and 30.4 g butyl acrylate. The kettle was then heated to 75—80°C. While the kettle was being heated to the final temperature, a pre-emulsion was prepared which contained 11.75 g Igepal CO-630 sulfonate, 130.25 g water, 0.5 g potassium persulfate, 134.4 g methacrylic acid, and 89.6 g butyl acrylate. The pre-emulsion was placed in a pressure-equalizing addition funnel fitted with a mechanical stirrer. When the kettle temperature was reached, dropwise addition of the pre-emulsion was begun. Although addition was to proceed over 3 hours, the reaction was stopped after 30 minutes because of complete coagulation.

The latexes which can be produced can have in excess of 5% of an acidic monomer.

More particularly, monoethylenically unsaturated monocarboxylic acids such as acrylic acid, methacrylic acid, ethacrylic acid and crotonic acid, monoethylenically unsaturated dicarboxylic acids such as maleic acid, fumaric acid, itaconic acid and citraconic acid, and monoethylenically unsaturated tricarboxylic acids such as aconitic acid can be employed, as can their halogen-substituted (e.g., fluoro-chloro- and bromo- substituted) derivatives, e.g. -chloroacrylic acid, and the anhydrides of these acids, e.g., -chloroacrylic acid.

In the terpolymer examples, butyl acrylate has been specifically disclosed, but other polymerizable acrylate monomers, as for example methyl acrylate, ethyl acrylate, propyl acrylate, n-butyl acrylate, isobutyl acrylate, 2-ethylhexyl acrylate, methyl methacrylate, ethyl methacrylate, isobutyl methacrylate and 2-ethylhexyl methacrylate can be used. Other copolymerizable monomers which can be used include styrene and vinyl acetate.

It should also be noted that terpolymers are described by way of example and not by way of limitation and copolymers can also be employed.

Further copolymerizable monomers include derivatives of vinyl alcohol, e.g., aliphatic vinyl esters, the vinyl ester of Versatic acid, allyl esters of saturated monocarboxylic acids, aliphatic vinyl ethers, vinyl ketones, ethylenically unsaturated monocarboxylic and polycarboxylic, as well as the available anhydrides, nitriles, unsubstituted and substituted amides of said acids can be used. A more detailed list of monomers is found in US—A—3,637,563, the relevant disclosure of which is incorporated herein by reference.

The sulfonate surfactants enable the emulsion to remain stable over extended periods of time. The percent of vinylic acid which can be present without encountering a stabilization problem increases with decreasing percent solids in the emulsion. Thus, while at 60% acid content and 40% solids coagulation occurs, decreasing either or both the acid content or solids levels, produces the desired coagulation free emulsion.

The concentration of the surfactant in the system is not narrowly critical and generally, a surfactant concentration range from 0.1 to 10 percent by weight of total monomer concentration gives the desired result.

Analytical techniques

a. Coagulum—Solids which were held in the 0.025 mm screen are thoroughly washed, dried in a 110°C oven for two hours and weighed. When the amount of solid present is too small to collect, or if the coagulum is water soluble, a qualitative description is recorded.

b. Mechanical Stability—The filtered latex is placed in an Osterizer blender, and blended for 10 minutes at maximum speed. If the latex coagulates during this time, mechanical stability is no good.

c. Brookfield Viscosity—The filtered latex sample is poured into a 1/4 l sample bottle. The viscometer is fitted with the proper spindle, set for the proper rpm, and lowered into the latex. The viscosity is read directly from a dial.

d. % Solids—Three weighing pans are tared, about 5 g. of the latex is placed in each, and the samples are placed in a 110—120°C oven for 2 hours. The dried sample is then weighed, and % solids is calculated from (dry weight/wet weight)×100.

e. Surface tension is measured with a Fisher Tensiomat.

f. pH is measured with any available pH meter.

g. Turbidity—A sample of the latex is placed in a test tube and diluted with a 1% solution of sodium lauryl sulfate to give an absorbance reading of between 0.5 and 0.8 at 450 nm in a Spectronic 20 visible spectrometer. Absorbance readings are made at 450, 500, 550 and 600 nm. The slope of a best fit straight line for the relationship of log (absorbance) versus log (wavelength) is determined with a least square computer program. This slope is recorded as turbidity.

TABLE I

| Surfactant | Butyl acrylate | Styrene | Methacrylic acid | Coagulum | Mechanical stability | Brookfield viscosity | % Solids | Turbidity | pH | Surface tension |
|---|---|---|---|---|---|---|---|---|---|---|
| Igepal CO-630—Sulfonate | 50% | 25% | 25% | V. Slight | OK | 29 (1—60) | 42.9 | 3.78 | 3.3 | 48.4 |
| Igepal CO-630—Sulfonate | 30% | 30% | 40% | 0.2% | OK | 7500 (2—3) | 50.2 | 3.03 | 3.2 | 68.0 |
| Alfol 1012 & 3 E.O. Sulfonate | 50% | 25% | 25% | 1.9% | OK | 11 (1—60) | 39.6 | 1.77 | 2.6 | 42.0 |
| Igėpal CO-630—Sulfonate*** | 40% | 0% | 60% | 100% | — | — | — | — | — | — |
| (Oxotridecyl & 4.2 E.O.) Sulfate*** | 50% | 25% | 25% | Foam** | OK | 120 (1—30) | 40.9 | 2.11 | 2.9 | 52.0 |
| | 50% | 25% | 25% | Foam** | OK | 104 (1—30) | 43.3 | 2.06 | 3.0 | 48.0 |
| (Oxotridecyl & 4.2 E.O.) Sulfonate | 50% | 25% | 25% | Foam | OK | 25 (1—60) | 41.5 | 3.29 | 2.8 | 43.0 |
| (Oxotridecyl & 4.2 E.O.) Sulfonate | 50% | 25% | 25% | Foam | OK | 32 (1—60) | 41.0 | 3.34 | 2.75 | 39.0 |
| (Alfol 1012 & 3 E.O.) Sulfonate | 50% | 25% | 25% | 7.5 gm | OK | 20 (1—60) | 40.9 | 1.81 | 2.6 | 45.0 |
| (Igepal 850)—Sulfonate | 50% | 25% | 25% | Foam | OK | 21 (1—60) | 42.9 | 3.53 | 2.75 | 53.0 |
| | 50% | 25% | 25% | Foam | OK | 26 (1—60) | 40.5 | 3.44 | 2.8 | 48.0 |
| (Alfol 12 & 4 E.O.) Sulfonate | 50% | 25% | 25% | Foam | OK | 40 (1—60) | 41.2 | 2.91 | 2.75 | 43.0 |
| (Alfol 12 & 4 E.O.) Sulfonate | 50% | 25% | 25% | V. Slight | OK | 44 (1—60) | 40.6 | 3.01 | 2.65 | 42.8 |

** Complete Coagulation after 3 days
* Coagulated in 24 hours
*** Comparison

## Claims

1. An aqueous emulsion comprising the reaction product of a vinyl acid monomer and at least a second copolymerizable monomer, said vinyl acid monomer being present in an amount equal to 5% to 50% by weight based on total weight of said vinyl acid monomer and said second copolymerizable monomer and a surfactant, said surfactant being present in an amount equal to 0.1 to 10 weight percent based on the total weight of said monomers, said surfactant being represented by the formula:

$$RO(CH_2CH_2O)_{n-1}CH_2CH_2SO_3Me$$

wherein:

R is an alkyl having 10 to 14 carbon atoms or a phenyl group, which is substituted by an alkyl group of 6 to 18 carbon atoms or 2 alkyl groups in which the first alkyl group contains 6 to 18 carbon atoms and the second alkyl group contains 6 to 12 carbon atoms;

n is at least 3; and

Me is $NH_4$, Na, Li or K, with the proviso that Me=Na when R is an alkyl group with 10 to 14 carbon atoms.

2. The aqueous emulsion of claim 1, wherein said second monomer comprises from 95% to 50% (based on the total of monomers) of at least one monomer selected from acrylates and/or styrene.

3. The aqueous emulsion of any of the preceding claims, wherein n is from 6 to 20.

4. The aqueous emulsion of any of the preceding claims, wherein said vinyl acid monomer contains acrylic acid.

5. The method of producing a stable aqueous emulsion according to any of claims 1 to 4 characterized that the said monomers and the said surfactant are contacted in an aqueous solution at an elevated temperature for a time sufficient to copolymerize said monomers.

## Patentansprüche

1. Wässrige Emulsion, enthaltend das Reaktionsprodukt eines Vinylsäuremonomeren und mindestens eines zweiten copolymerisierbaren Monomeren, wobei das Vinylsäuremonomere in einer Menge von 5 bis 50 Gew.-%, bezogen auf das Gesamtgewicht aus dem Vinylsäuremonomeren und dem zweiten copolymerisierbaren Monomeren, anwesend ist, und ein oberflächenaktives Mittel, das in einer Menge von 0,1 bis 10 Gew.-%, bezogen auf das Gesamtgewicht dieser Monomeren, anwesend ist und durch die Formel

$$RO(CH_2CH_2O)_{n-1}CH_2CH_2SO_3Me$$

dargestellt wird, in welcher

R eine Alkylgruppe mit 10 bis 14 Kohlenstoffatomen oder eine Phenylgruppe ist, die durch eine Alkylgruppe mit 6 bis 18 Kohlenstoffatomen oder 3 Alkylgruppen substituiert sein kann, wobei die erste Alkylgruppe 6 bis 18 Kohlenstoffatome und die zweite Alkylgruppe 6 bis 12 Kohlenstoffatome enthält;

n mindestens 3 ist; und

Me $NH_4$, Na, Li oder K ist, mit der Bedingung, daß Me=Na ist, wenn R eine Alkylgruppe mit 10 bis 14 Kohlenstoffatomen ist.

2. Wässrige Emulsion nach Anspruch 1, in welcher das zweite Monomere 95 bis 50% (bezogen auf die Gesamtmenge der Monomeren) mindestens eines Monomeren, ausgewählt aus Acrylaten und/oder Styrol, enthält.

3. Wässrige Emulsion nach einem der vorhergehenden Ansprüche, in welcher n 6 bis 20 ist.

4. Wässrige Emulsion nach einem der vorhergehenden Ansprüche, in welcher das Vinylsäuremonomere Acrylsäure enthält.

5. Verfahren zur Herstellung einer stabilen wässrigen Emulsion gemäß einem der Ansprüche 1 bis 4, dadurch gekennzeichnet, daß diese Monomeren und das oberflächenaktive Mittel in einer wässrigen Lösung bei erhöhter Temperatur für eine zum Copolymerisieren der Monomeren ausreichende Zeit in Berührung gebracht werden.

## Revendications

1. Emulsion aqueuse comprenant le produit de réaction d'un monomère acide à groupe vinyle et d'au moins un second monomère copolymérisable, ledit monomère acide à groupe vinyle étant présent en une quantité égale à 5—50% en poids sur la base du poids total dudit monomère acide à groupe vinyle et dudit second monomère copolymérisable, et un surfactif, le surfactif étant présent en une quantité égale à 0,1—10% en poids sur la base du poids total des monomères, ledit surfactif étant représenté par la formule:

$$RO(CH_2CH_2O)_{n-1}CH_2CH_2SO_3Me$$

dans laquelle

R est un groupe alkyle ayant 10 à 14 atomes de carbone ou un groupe phényle qui est substitué par un groupe alkyle de 6 à 18 atomes de carbone ou par 2 groupes alkyle dont le premier contient 6 à 18 atomes de carbone et le second contient 6 à 12 atomes de carbone;

n est égal à 3 au moins; et

Me représente $NH_4$, Na, Li ou K, sous réserve que Me soit égal à Na lorsque R est un groupe alkyle ayant 10 à 14 atomes de carbone.

2. Emulsion aqueuse suivant la revendication 1, dans laquelle le second monomère comprend 95 à 50% (sur la base du poids total des monomères) d'au moins un monomère choisi entre les acrylates et/ou le styrène.

3. Emulsion aqueuse suivant l'une quelconque des revendications précédentes, dans laquelle n a une valeur de 6 à 20.

4. Emulsion aqueuse suivant l'une quelconque des revendications précédentes, dans laquelle ledit monomère acide à groupe vinyle contient de l'acide acrylique.

5. Procédé de production d'une émulsion aqueuse stable suivant l'une quelconque des revendications 1 à 4, caractérisé en ce que lesdits monomères et ledit surfactif sont mis en contact en solution aqueuse à une température élevée pendant une période suffisante pour copolymériser lesdits monomères.